(19) Europäisches Patentamt
European Patent Office
Office européen des brevets

(11) **EP 2 055 280 A2**

(12) **EUROPEAN PATENT APPLICATION**

(43) Date of publication:
06.05.2009 Bulletin 2009/19

(51) Int Cl.:
*A61F 13/532* (2006.01)    *A61F 13/537* (2006.01)

(21) Application number: 08253526.1

(22) Date of filing: 29.10.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 30.10.2007 US 983780 P
17.12.2007 US 957969

(71) Applicant: McNeil-PPC, Inc.
Skillman, NJ 08558 (US)

(72) Inventors:
• Bissah, Kofi Ayensu
Somerset, NJ 08873 (US)
• De Oliveira, Richardo
New Hope, PA 18938 (US)
• Tremblay, Denis
Pointe-aux-Trembles, Quebec H1A 4M5 (CA)

(74) Representative: Kirsch, Susan Edith et al
Carpmaels & Ransford
43-45 Bloomsbury Square
London WC1A 2RA (GB)

(54) **Absorbent article including an absorbent layer having a plurality of spaced beam elements**

(57) The present invention generally relates to absorbent sanitary napkins and in particular to a sanitary napkin including an absorbent layer having a plurality of spaced beam elements for promoting enhanced fluid wicking within the sanitary napkin.

**EP 2 055 280 A2**

Description

CROSS REFERENCE TO RELATED APPLICATIONS

This application claims priority to Application No. 60/983,780 filed on October 30, 2007, the entire contents of which are incorporated by reference herein.

FIELD OF INVENTION

The present invention generally relates to absorbent sanitary napkins and in particular to a sanitary napkin including an absorbent layer having a plurality of spaced beam elements for promoting enhanced fluid wicking within the sanitary napkin.

BACKGROUND OF THE INVENTION

In order for a sanitary napkin to efficiently absorb a large amount of fluid during use it must effectively wick fluid throughout the absorbent structure of the napkin. Absent effective wicking properties menstrual fluid tends to pool in certain regions of the napkin as a result of which the full absorbent capacity of the napkin is not effectively utilized. On the other hand, napkins that exhibit particularly efficient wicking characteristics may exhibit other problems. In particular, such napkins may wick fluid to the longitudinal edge of the absorbent structure resulting in fluid being released from the longitudinal edge of the napkin, i.e. resulting in side leakage. Accordingly, the inventors of the present invention have recognized a need to provide a sanitary napkin that efficiently wicks fluid in the longitudinal direction and at the same time effectively prevents side leakage in the transverse direction of the napkin. By providing a napkin that efficiently wicks fluid in the longitudinal direction and at the same time effectively prevents side leakage in the transverse direction, the inventor's have provided a sanitary napkin that exhibits superior fluid handling characteristics and effectively utilizes the full absorbent capacity of the napkin.

SUMMARY OF THE INVENTION

In view of the foregoing, the present invention provides, according to a first aspect of the invention an absorbent article including a longitudinally extending centerline, a transversely extending centerline, a cover layer having a body facing surface, a first absorbent layer having an upper surface and a lower surface, the layer comprising a plurality of beams and a plurality of material-free zones, each of the beams arranged in a spaced relationship to an adjacent beam and each of the beams being separated from an adjacent beam by a material-free zone, each of the material-free zones extending from the upper surface to the lower surface, a second absorbent structure arranged below the first absorbent layer, wherein the cover layer includes a plurality of first regions arranged in spaced relationship to the second absorbent structure and a plurality of second regions, each of the second regions located between two adjacent beams and arranged in surface to surface contact with the second absorbent structure, wherein the first absorbent layer, second absorbent layer and cover cooperate to define a plurality of longitudinally extending gutters in the body facing surface of the article, wherein the absorbent article has a thickness of less than 4.0 mm, and wherein the absorbent article has an RIF value greater than 2.6.
The present invention provides, according to a second aspect of the invention, an absorbent article including a cover layer having a body facing surface, a first absorbent layer having an upper surface and a lower surface, the layer comprising a plurality of material-free zones, each of the material-free zones extending from the upper surface to the lower surface, a second absorbent structure arranged below the first absorbent system, wherein the cover layer includes a plurality of first regions arranged in spaced relationship to the second absorbent structure and a plurality of second regions, each of the second regions arranged in surface to surface contact with the second absorbent structure, wherein the absorbent article has a thickness of less than 4.0 mm, and wherein the absorbent article has an LTWR value greater than 2.4.

BRIEF DESCRIPTION OF THE DRAWINGS

Examples of embodiments of the present invention will now be described with reference to the drawings, in which:

Fig. 1 is a top plan view of the absorbent article according to the present invention;
Fig. 2 is a bottom plan view of the absorbent article shown in Fig. 1;
Fig. 3a is an exploded view of the absorbent article shown in Fig. 1 according to a first embodiment of the invention;
Fig. 3b is an exploded view of the absorbent article shown in Fig. 1 according to a second embodiment of the invention;

Fig. 4 is an exploded view of the second absorbent layer employed in the absorbent article shown in Fig. 1;

Fig. 5 is a sectional view of the absorbent article shown in Fig. 1 taken along line 5-5 thereof;

Fig. 6 is a perspective view of an absorbent article according to a second embodiment of the present invention;

Fig. 7 is a top elevation view of a test plate used to measure a Longitudinal/Transverse Wicking Ratio of an absorbent article and a Repeat Insult Failure (RIF) Value of an absorbent article; and

Fig. 8 is a schematic view depicting a stained absorbent article and the manner in which the Longitudinal/Transverse Wicking Ratio is calculated.

## DETAILED DESCRITION OF THE INVENTION

The present invention generally relates to disposable absorbent articles such as sanitary napkins, pantiliners, absorbent products for incontinence, and other disposable absorbent articles worn close to a wearer's body. Although the invention will be described herein with reference to a sanitary napkin 10 the invention may be utilized with other disposable sanitary absorbent articles such as absorbent products for incontinence, diapers, pantiliners and the like.

Absorbent articles according to the present invention provide superior fluid handling characteristics, and more specifically are particularly adept at preventing side leakage while at the same time providing superior longitudinal wicking characteristics.

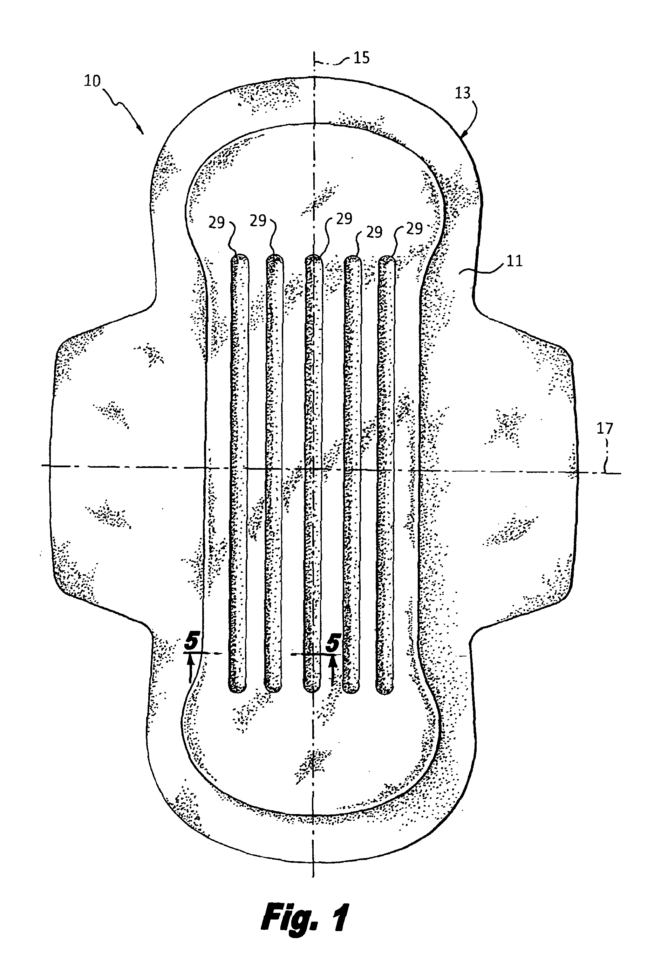

As shown in Figs. 1-5, the present invention according to one embodiment of the invention relates to a sanitary napkin 10 for absorbing bodily fluids. The sanitary napkin 10 includes a body facing surface 11, a garment facing surface 13, a longitudinally extending centerline 15, and a transversely extending centerline 17.

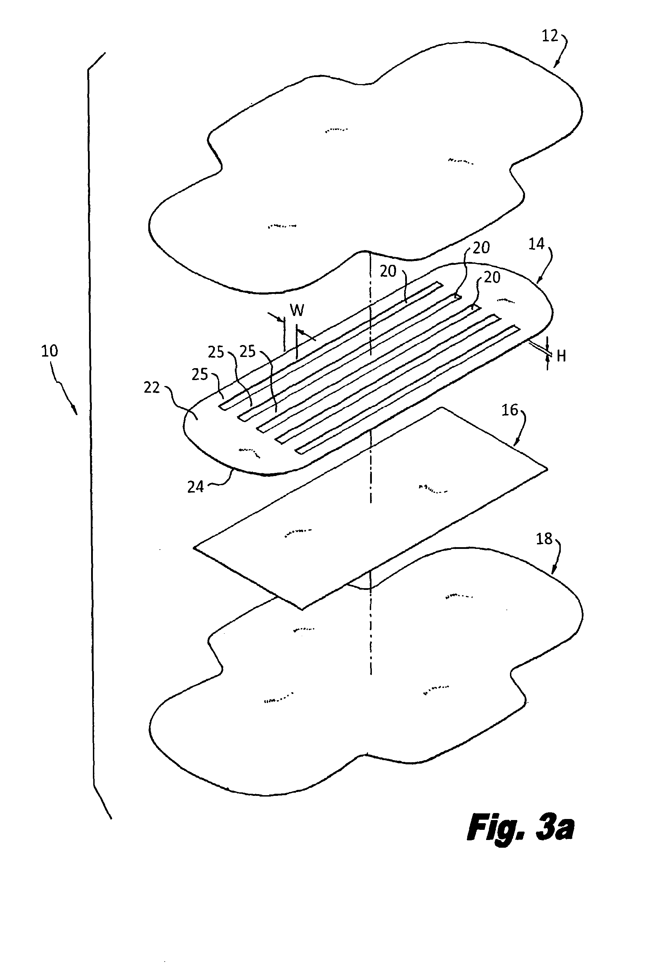

As best seen in the exploded view shown in Fig. 3a, the sanitary napkin 10 includes a fluid permeable cover layer 12, a first absorbent layer 14, a second absorbent layer 16, and a fluid impermeable barrier layer 18. The first absorbent layer 14 includes a plurality of longitudinally extending material-free zones 20 that extend from an upper surface 22 of the first absorbent layer 14 to a lower surface 24 of the first absorbent layer 14. Each of the material-free zones 20 preferably has a width in the range of between 1 mm and about 10 mm and a length in the range of between about 5 cm and about 40 cm. Absorbent articles according to the present invention preferably have between about 1 and about 7 longitudinally extending the material-free zones 20. Each of the material free zones 20 is spaced from an adjacent material-free zone 20 in the transverse direction by a distance from about 0.5 cm to about 3 cm. The material-free zones 20 preferably extend over a surface area in the range of between 50 mm$^2$ and about 4000 mm$^2$. In the particular embodiment of the invention shown in the Figures the material-free zones 20 are linear in shape, parallel to each other, and equally spaced.

The first absorbent layer 14 further includes a plurality of longitudinally extending beams 25, each of the beams 25 being arranged in spaced relationship to an adjacent beam 25 and each of the beams 25 being separated from an adjacent beam 25 by one of the material-free zones 20.

In one embodiment of the invention, as shown in Fig. 3a, each beam 25 is structured and arranged such that it has a height less than its width, in other words H<W. Also, in this particular embodiment of the invention, each beam 25 is structured and arranged such that a ratio of its height to width is less than one, in other words H/W < 1.

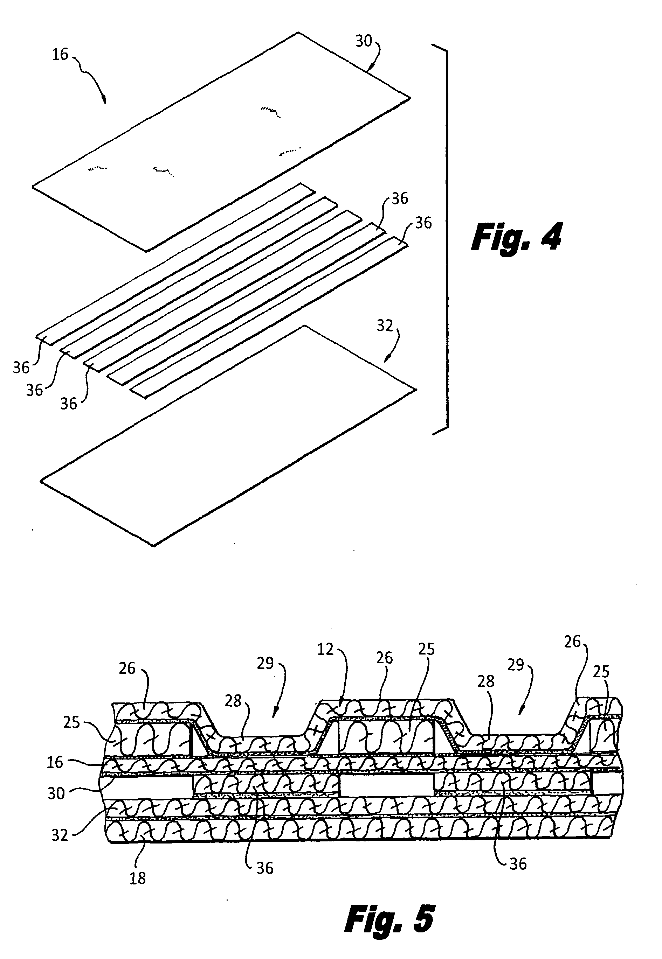

As best seen in Fig. 5, the cover layer 12 includes a plurality of first regions 26 that are arranged in spaced relationship to the second absorbent structure 16 and a plurality of second regions 28 that are arranged in surface to surface contact with the second absorbent structure 16.

The surface to surface contact of the cover layer 12 with the second absorbent structure in the second regions 28 essentially define a plurality of longitudinally extending gutters 29 in the body facing surface 11 of the napkin 10 that are coextensive with the path of the material-free zones 20. The depth of each gutter 29 is equivalent to the thickness of the first absorbent layer 14. The first absorbent layer 14 preferably has a thickness of between about 0.5 mm and about 3 mm, thus the depth of each gutter 29 is in the range of between about 0.5 mm and about 3 mm. The thickness and depth measurements set forth in this paragraph may be determined by using a suitable thickness gauge such as the Mitutoyo Absolute Gauge or equivalent.

In a preferred embodiment of the invention, shown in Fig. 4, the second absorbent layer 16 is a multilayer construct including a first absorbent layer 30, a second absorbent layer 32, and superabsorbent material arranged between the first 30 and second layers 32. In the particular embodiment shown in Fig. 4 the superabsorbent material is arranged in a plurality of spaced lines 36. Preferably each of the spaced lines 36 of superabsorbent material is positioned such that it is arranged in vertical alignment with one of the gutters 29.

Although not shown in the Figures, the areas of the napkin 10 in which the gutters 29 are located may be colored a different color than the remainder of the absorbent article. For example, the areas in which the gutters 29 are located may be colored blue while the remainder of the napkin 10 is generally white. By coloring the gutters 29 a different color than the remainder of the napkin 10 the enhanced wicking characteristics provided by the gutters 29 are visually communicated to a potential user of the absorbent article. The color may be imparted to the napkin 10 by providing a color (e.g., ink) to the cover layer 12 and/or the second absorbent layer 16 and/or the barrier layer 18.

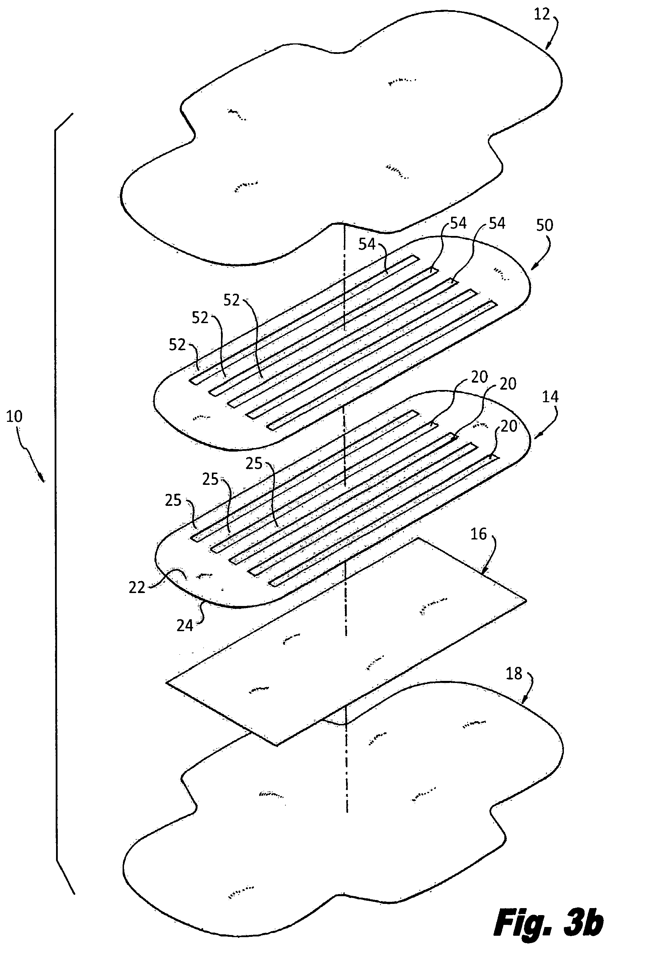

Fig. 3b shows another embodiment of a sanitary napkin 10b according to the present invention. The sanitary napkin 10b further includes a transfer layer 50 arranged between the cover layer 12 and the first absorbent layer 14. The transfer layer 50 preferably includes a plurality of beams 52 and a plurality of material-free zones 54, each one of the material-free zones 54 is arranged between adjacent beams 52. Each of the beams 52 of the transfer layer 50 are preferably arranged in vertical alignment with a corresponding beam 25 of the first absorbent layer 14 and each of the material-free zones 54 of the of the transfer layer 50 is preferably arranged in vertical alignment with a corresponding material-free zone 20 of the first absorbent layer 14. The transfer layer 50 may be constructed from similar materials as those described below for the first absorbent layer 14.

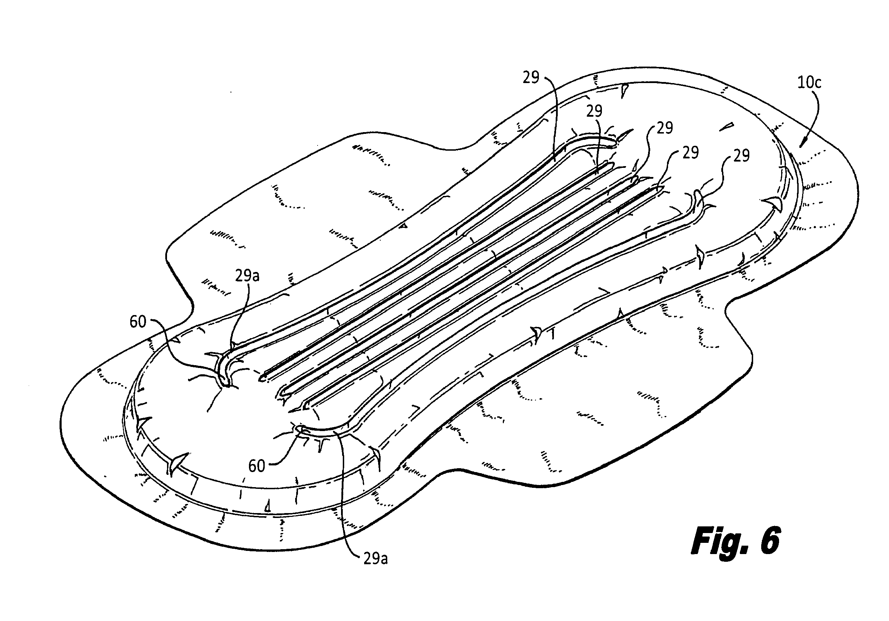

Figure 6 shows another embodiment of a sanitary napkin 10c according to the present invention. In the sanitary napkin 10c the gutters 29 extend substantially in the longitudinal direction of the napkin, however the outermost gutters 29a in the transverse direction have curved end portions 60 that extend in a transverse direction toward the longitudinally extending centerline 15 of the article.

Cover Layer

The cover layer 12 may be a relatively low density, bulky, high-loft non-woven web material. The cover layer 12 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 12 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 12 preferably has a relatively high degree of wettability, although the individual fibers comprising the cover may not be particularly hydrophilic. The cover material should also contain a great number of relatively large pores. This is because the cover layer 12 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 12 should not lose their physical properties when they are wetted, in other words they should not collapse or lose their resiliency when subjected to water or body fluid. The cover layer 12 may be treated to allow fluid to pass through it readily. The cover layer 12 also functions to transfer the fluid quickly to the underlying layers of the napkin. Thus, the cover layer 12 is advantageously wettable, hydrophilic and porous. When composed of synthetic hydrophobic fibers such as polyester or bi-component fibers, the cover layer 12 may be treated with a surfactant to impart the desired degree of wettability.

Alternatively, the cover layer 12 can also be made of polymer film having large pores. Because of such high porosity, the film accomplishes the function of quickly transferring body fluid to the inner layers of the underlying absorbent layers. A suitable cover material of this type is commercially found on the STAYFREE Dry Max Ultrathin product distributed by McNeil-PPC, Inc.

The cover layer 12 may be attached to the underlying absorbent layers 14 and 16, and/or the barrier layer 18, by adhesion and/or other suitable means know to those of skill in the art.

First Absorbent Layer

The first absorbent layer 14 may be composed of fibrous materials, such as wood pulp, polyester, rayon, flexible foam, or the like, or combinations thereof. The first absorbent layer 14 may also optionally include a superabsorbent polymer (SAP) material. The first absorbent layer 14 may also comprise thermoplastic fibers for the purpose of stabilizing the layer and maintaining its structural integrity. The first absorbent layer 14 may be treated with surfactant on one or both sides in order to increase its wettability, although generally the first absorbent layer 14 is relatively hydrophilic and may not require treatment. The first absorbent layer 14 is preferably bonded on both sides to the adjacent layers, i.e. the cover layer 12 and the underlying second absorbent layer 16.

In one specific embodiment of the invention the first absorbent layer 14 is a through air bonded pulp material commercially available from Buckeye Technologies, Memphis, Tenn. under the designation VIZORB 3045.

Second Absorbent Layer

The second absorbent layer 16 may comprise a single layer of material or may comprise multiple layers.

In one embodiment, the second absorbent layer 16 is a blend or mixture of cellulosic fibers and superabsorbent disposed therein. Cellulosic fibers that can be used in the second absorbent layer 16 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The second absorbent layer 16 can contain any superabsorbent polymer (SAP) which are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc. In a specific example, the second absorbent layer 16 is a material containing from 90% to about 40% percent cellulosic fiber, about 10% to about 60% SAP. The second absorbent layer 16 may comprise a material manufactured by using air-laying means well known in the art.

In one preferred embodiment of the invention the second absorbent layer 16 is relatively thin, high swelling absorbent material such as the absorbent composite material sold under the trade name NOVATHIN ® available from EAM Corporation located in Jessup, Ga. , U.S.A.

In a preferred embodiment of the invention, shown in Fig. 4, the second absorbent layer 16 is a multilayer construct including a first absorbent layer 30, a second absorbent layer 32, and superabsorbent material 34 arranged between the first 30 and second layers 32. In the particular embodiment shown in Fig. 4 the superabsorbent material 34 is arranged in a plurality of spaced lines 36. The first absorbent layer 30 and second absorbent layer 32 are constructed from pulp tissue commercially available from Little Rapids Corporation, Green Bay, Wisconsin and the superabsorbent material 34 is a superabsorbent polymer commercially available as Aqua Keep BA40B from Sumitomo Seika Chemicals Co., Ltd., Osaka, Japan.

Barrier Layer

Underlying the second absorbent layer 16 is a barrier layer 18 comprising liquidimpervious film material so as to prevent liquid that is entrapped in the absorbent layer 16 from egressing the sanitary napkin and staining the wearer's undergarment. The barrier layer 18 is preferably made of polymeric film, although it may be made of liquid impervious, air-permeable material such as repellent-treated non-woven or micropore films or foams.

The barrier layer 18 may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 12 and the barrier layer 18 are preferably joined along their marginal portions so as to form an enclosure or flange seal that maintains the absorbent layers 14 and 16 captive. The joint may be made by means of adhesives, heat-bonding, ultrasonic bonding, radio frequency sealing, mechanical crimping, and the like and combinations thereof.

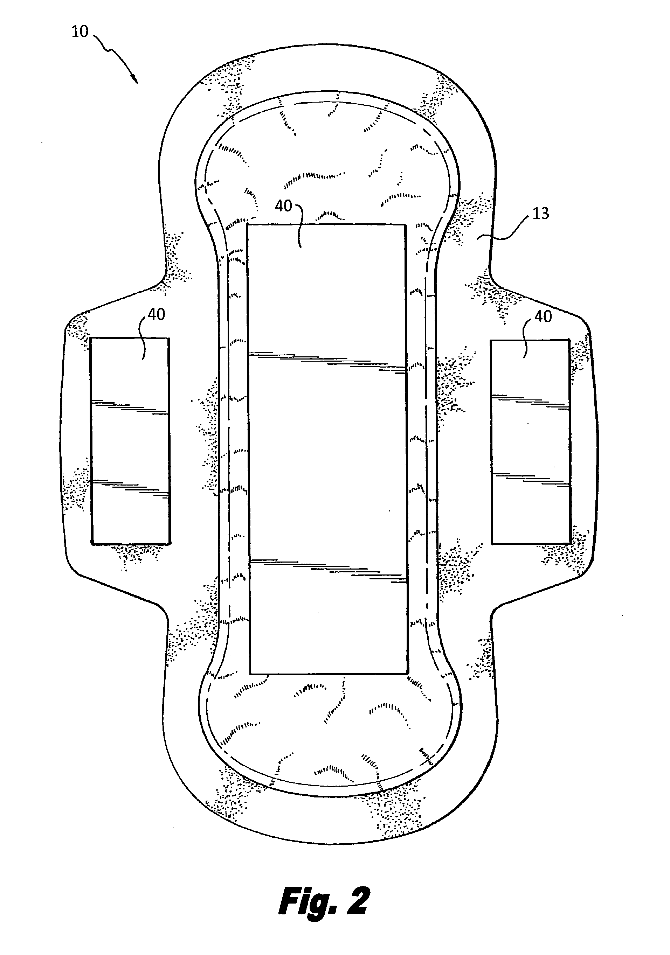

Positioning adhesive may be applied to a garment facing surface 13 of the barrier layer 18 for securing the napkin 10 to a garment during use. As seen in Fig. 2, the positioning adhesive may be covered with removable release paper 40 so that the positioning adhesive is covered by the removable release paper 40 prior to use.

Absorbent articles of this invention may or may not include wings, flaps or tabs for securing the absorbent article to an undergarment. Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

Test Procedures

Absorbent articles according to the present invention possess a combination of unique functional properties.

The test procedures set forth below highlight each of these functional properties. Prior to conducting any of the described test procedures described below the test product should be conditioned for two hours at 21 +/- 1° C and 50 +/- 2 % humidity.

Procedure for Measuring the Thickness of Absorbent Article

Preferred embodiments of the present invention relate to "ultra-thin" sanitary napkins. "Ultra-thin" sanitary napkins as defined herein are those sanitary napkins that have a thickness of less than 4.0 mm, according to the thickness measurement procedure set forth below. Preferably, absorbent articles according to the present invention have a thickness in the range of about 1.5 mm to about 3.0 mm.

The apparatus required to measure the thickness of an absorbent article is a footed dial (thickness) gauge with stand, available from Ames, with a 2" (5.08 cm) diameter foot at a pressure of 0.07 psig and a readout accurate to 0.001" (0.00254 cm). A digital type apparatus is preferred. If the absorbent article sample is individually folded and wrapped, the sample is unwrapped and carefully flattened by hand. The release paper is removed from the product sample and it is repositioned back gently across the positioning adhesive lines so as not to compress the sample, ensuring that the release paper lies flat across the sample. Flaps (if any) are not considered when taking the thickness.

The foot of the gauge is raised and the product sample is placed on the anvil such that the foot of the gauge is approximately centered over the intersection of the longitudinally extending centerline and transversely extending centerline on the product sample. When lowering the foot, care must be taken to prevent the foot from dropping onto the sample or from undue force being applied. A load of 0.07 p.s.i.g. is applied to the sample and the read out is allowed to stabilize for approximately 5 seconds. The thickness reading is then taken. This procedure is repeated for at least five product samples and the average thickness is then calculated.

Procedure for Measuring Modified Circular Bend Stiffness (MCB)

Absorbent articles according to the present invention exhibit increased multi-directional flexibility when compared to comparable prior art absorbent articles. The test method set forth below provides a method for measuring multi-directional flexibility of an absorbent article. Absorbent articles according to the present invention preferably have an MCB stiffness of less than 400 g, more preferably less than 350 g, and most preferably less than 300 g.

Modified Circular Bend Stiffness (MCB) is determined by a test that is modeled after the ASTM D 4032-82 CIRCULAR BEND PROCEDURE, the procedure being considerably modified and performed as follows. The CIRCULAR BEND PROCEDURE is a simultaneous multi-directional deformation of a material in which one face of a specimen becomes concave and the other face becomes convex. The CIRCULAR BEND PROCEDURE gives a force value related to flexural resistance, simultaneously averaging stiffness in all directions.

The apparatus necessary for the CIRCULAR BEND PROCEDURE is a modified Circular Bend Stiffness Tester, having the following parts:

1. A smooth-polished steel plate platform, which is 102.0 mm by 102.0 mm by 6.35 mm having an 18.75 mm diameter orifice. The lap edge of the orifice should be at a 45 degree angle to a depth of 4.75 mm;
2. A plunger having an overall length of 72.2 mm, a diameter of 6.25 mm, a ball nose having a radius of 2.97 mm and a needle-point extending 0.88 mm therefrom having a 0.33 mm base diameter and a point having a radius of less than 0.5 mm, the plunger being mounted concentric with the orifice and having equal clearance on all sides. Note that the needle-point is merely to prevent lateral movement of the test specimen during testing. Therefore, if the needle-point significantly adversely affects the test specimen (for example, punctures an inflatable structure), than the needle-point should not be used. The bottom of the plunger should be set well above the top of the orifice plate. From this position, the downward stroke of the ball nose is to the exact bottom of the plate orifice;
3. A force-measurement gauge and more specifically an Instron inverted compression load cell. The load cell has a load range of from about 0.0 to about 2000.0 g;
4. An actuator and more specifically the Instron Model No. 1122 having an inverted compression load cell. The Instron 1122 is made by the Instron Engineering Corporation, Canton, Mass.

In order to perform the procedure for this test, as explained below, five representative product samples for each article to be tested are necessary. The location of the sanitary napkin, or other absorbent article, to be tested is located at the intersection of the longitudinally extending centerline and transversely extending centerline of the absorbent article. A 37.5 mm by 37.5 mm test specimen is cut from each of the five product samples. Prior to cutting the test specimens any release paper or packaging material is removed from the product sample and any exposed adhesive, such as garment positioning adhesive, is covered with a non-tacky powder such as talc or the like. The talc should not affect the MCB measurement.

The test specimens should not be folded or bent by the test person, and the handling of specimens must be kept to a minimum and to the edges to avoid affecting flexural-resistance properties.

A test specimen is centered on the orifice platform below the plunger such that the body facing layer of the test specimen is facing the plunger and the barrier layer of the specimen is facing the platform. The plunger speed is set at 50.0 cm per minute per full stroke length. The indicator zero is checked and adjusted, if necessary. The plunger is actuated. Touching the test specimen during the testing should be avoided. The maximum force reading to the nearest gram is recorded. The above steps are repeated until all five test specimens have been tested. An average is then taken from the five test values recorded to provide an average MCB stiffness.

Procedure for Measuring Longitudinal Transverse Wicking Ratio (LTWR)

Absorbent articles according to the present invention exhibit superior wicking in the longitudinal direction of the article yet at the same time control wicking in the transverse direction of the article to thereby prevent side leakage. The Longitudinal Transverse Wicking Ratio (LTWR) test set forth below illustrates the superior wicking characteristics of absorbent articles according to the present invention.

Absorbent articles according to the present invention have LTWR values greater than 1.5, more preferably greater than 2.0, and most preferably greater than 2.4.

The synthetic test fluid described below is used in the test method described below. The synthetic test fluid used in replacement of human menses due to its ease in preparation and accessibility of the ingredients.

The fluid is prepared by dissolving each of the following components into distilled water. Care should be taken to ensure that components are well dissolved. A rotating blade mixer or a magnetic stirrer should be used for mixing the components. In a large enough container, add the following components, making sure that the component is dissolved before adding the next one:

| Quantity/1L | Reagent | Grade, purity | Supplier | Catalog no. |
|---|---|---|---|---|
| 9.0 g | sodium chloride | ACS reagent 99+% | Sigma-Aldrich | 223514 |
| 490.5 g | distilled water | N/AP | N/AP | N/AP |
| 10 g | 2-phenoxyethanol | puriss. 99.0% | Sigma-Aldrich (Fluka) | 77699 |
| 0.5 g | FD&C Red #40 | Food | A&C | C3465 |
| 490.5 g | glycerol | ACS reagent 99.5% | Sigma-Aldrich | G7893 |

The materials required to determine the LTWR value including the following:

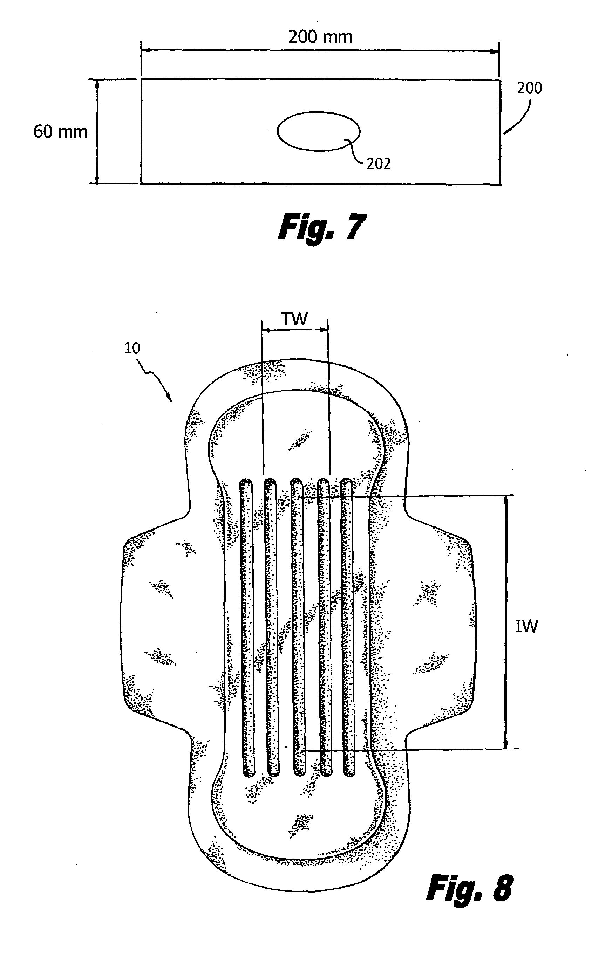

- a poly(methylmetracrylate) (Plexiglas) template 200, shown in Fig. 7, having dimensions of 20 cm long x 6 cm wide x 1.27 cm thick and an elliptical orifice 202 (3.8 cm x 1.9 cm) in the center of the template;
- a calibrated electronic repeater pipette (HandyStep Electronic Repeating Pipet, Brandtech) with a 50 mL combi-syringe (or combi-tip) capable of delivering 5-10 mL at a rate of approximately 4 mL/s and fixed on a stand with the end of the tip placed vertically at 2.54 cm (1 inch) from the surface of the product;
- a calibrated stopwatch that has a precision of 0.01 s;
- a ruler graduated in millimeters (mm);
- a fine point permanent marker; and
- the test fluid prepared according to the procedure described above.

To obtain the LTWR value, the conditioned article is removed from its packaging, unfolded, placed on a flat surface (e.g. lab table). The Plexiglas plate 200 is placed and centered over the absorbent product, light hand applied pressure is applied to flatten the article, without compressing it, such that there is no substantial bending or folding of the article under the template 200.

A 50 mL combi-syringe (or combi-tip), placed on a repeater pipette, is filled with the test fluid, positioned vertically and fixed on a stand with the end of the tip placed approximately 1 inch from the surface of the product and above the center of the elliptical hole 202 of the plate. The article should be arranged such that the intersection of the longitudinally extending and transversely extending centerlines is positioned in the center of the hole 202. Then, 7 mL of test fluid is insulted to the article at a rate of approximately 4 mL/s. The stopwatch is started as soon as the repeating pipet button is pressed to dispense the volume. After 60 s, the plate is removed and the limits of the stain, along the longitudinally extending and transversely extending axes, are marked rapidly (within less than 10 s) with a marker. Only the continuous fluid stain limits found on the article cover are marked, fluid spots not continuous or linked to the rest of the fluid flow and/or observed in the lower layers of the absorbent product are not considered in the determination of the wicking distance. Using the furthest continuous points found along the longitudinally and transversely extending axes, a rectangle is drawn as shown in Fig. 8. The length of the rectangle measured in the longitudinal direction (LW) is divided by the width of the rectangle measured in the transversal direction (TW) to give the LTWR value:

$$LTW = LW/TW$$

where the Longitudinal Wicking Distance (LW) is defined as the farthest continuous fluid displacement along the longitudinally extending axis of the article; and the Transverse Wicking Distance (TW) is the farthest continuous fluid displacement along transversely extending axis. All distance values are measured with a ruler to a precision of 1 mm.

If the test fluid touches or runs over the edges of the plate 200 in the transverse direction within 60 s following the fluid injection, the sample is considered to have failed and a zero (0) LTWR value is attributed to that sample. If the absorbent article being tested is smaller than the test plate 200, then failure is defined as the moment when the fluid reaches the edges of the absorbent article in the transverse direction.

This procedure is repeated five times using five different samples of each type of absorbent article and an average LTWR value is calculated from these five repeats. If any of the five products fails, i.e. the fluid reaches the edges of the plate, then the reported average LTWR value is zero (0).

Procedure for Measuring Repeat Insult Failure (RIF) Value

The ability of an absorbent article to hold repeated insults of fluid without leaking is an indication that absorbent article can deliver superior protection to a user. Absorbent articles according to the present invention preferably have a Repeat Insult Failure Value greater than 2.6 more preferably greater than 2.75, and most preferably greater than 2.85.

In the Repeat Insult Failure Value test method described below, a product fails when fluid reaches the edges of the Plexiglas template 200 shown in Fig. 7, which is centered over the article during the test.

In order to determine the RIF value, the conditioned article is removed from its packaging, unfolded, placed on a flat surface (e.g. lab table). The Plexiglas template 200 is placed and centered the over the article to tested and light hand applied pressure is applied to the template 200 to flatten the article, without compressing it, such that there is no substantial bending or folding of the article under the template 200. The article should be arranged such that the intersection of the longitudinally extending and transversely extending centerlines is positioned in the center of the hole 202.

A 50 mL combi-syringe (or combi-tip), placed on a repeater pipette, is filled with the test fluid (disclosed above), positioned vertically and fixed on a stand with the end of the tip placed approximately 1 inch from the surface of the product and above the center of the elliptical hole of the plate. Then, 7 mL of test fluid is injected on the pad with the repeater pipette at a rate of approximately 4 mLs. The stopwatch is started at the same time as the pipette button is pressed. As soon as the cover can be observed through the fluid anywhere in the elliptical hole, the time is recorded. This time is defined as the penetration time (p-time). After approximately 30 s, a second 7 mL volume of test fluid is injected, and again, the p-time is recorded. Additional 7 mL insults and p-time measurements are carried out until the product fails. Sample failure is achieved when the test fluid reaches one of the edges of the Plexiglas plate 200. The above described procedure is repeated for five (5) samples of each article to be tested. For each type of article, determine the average volume at which the product failed, $V_{(failure)}$, and the average penetration time obtained when the product failed, p-time$_{(failure)}$. Then, the RIF value is calculated using the following equation:

$$RIF = \frac{V_{(failure)}}{p-time_{(failure)}}$$

## **EXAMPLES**

Inventive Example

An inventive example of a sanitary napkin according to the present invention was constructed as follows.

A cover layer was removed from a Stayfree Dry Max Ultrathin product, distributed by McNeil-PPC, Inc., and was laid on a conveyor belt and slot coated with 1.3 gsm of hot melt adhesive commercially available as NW 1023 ZP from Bostik Corporation located in Wauwatosa, WI. A 100 gsm first absorbent layer was constructed from a through air bonded pulp material commercially available from Buckeye Technologies, Memphis, Tenn, under the designation VIZORB 3045. The first absorbent layer was die cut to form five longitudinally extending material free zones as shown in Fig. 1. Each of the material free zones had a length of 150 mm a width of 5 mm and each material free zone was spaced in the transverse direction for an adjacent material free zone by a distance 10 mm. The first absorbent layer had a thickness of 2 mm. The second absorbent layer was constructed from 208 gsm absorbent composite material sold under the trade name NOVATHIN ® commercially available from EAM Corporation located in Jessup, Ga., U.S.A.. Hot melt adhesive was then slot coated onto the second absorbent structure on a top surface thereof in the amount of 6.3 gsm to bond the first absorbent layer to the second absorbent layer. A 0.9 mil polymeric film basksheet commercially available from Pliant Corporation, Schaumburg, III. was placed on the garment facing side of the second absorbent layer. The cover was depressed into the into the areas defined by the material free zones to thereby form longitudinally extending gutters in the surface of the napkin and the cover bonded was bonded to the second absorbent layer in the area of the gutters by means of heat and pressure. The final composite was crimped along the peripheral edge of the article and final cut.

Comparative Example

Always Ultrathin Regular with Wings

Each of the Inventive Example and Comparative Example were tested according to the test methods described above and the results thereof are summarized in Table 1 provided below.

**TABLE 1**

| (average of = 5) | Inventive Example | Comparative Example #1 |
|---|---|---|
| MCB center (g) | 290 | 338 |
| Repeat Insult Value (Volume/p-time) at failure (mL/s) | 3.0 | 2.6 |
| Longitudinal/Transverse Wicking Ratio (cm/cm) | 2.46 | 1.27 |
| Thickness (mm) | 2.48 | 2.77 |

As illustrated in the table provided above absorbent articles according to the present invention provide superior comfort and fluid handling characteristics when compared to comparable prior art articles.

**Claims**

1. An absorbent article comprising:

  a longitudinally extending centerline;
  a transversely extending centerline;
  a cover layer having a body facing surface;
  a first absorbent layer having an upper surface and a lower surface, the layer comprising a plurality of beams and a plurality of material-free zones, each of the beams arranged in a spaced relationship to an adjacent beam and each of the beams being separated from an adjacent beam by a material-free zone, each of the material-free zones extending from the upper surface to the lower surface;
  a second absorbent structure arranged below the first absorbent layer;

  wherein the cover layer includes a plurality of first regions arranged in spaced relationship to the second absorbent structure and a plurality of second regions, each of the second regions located between two adjacent beams and arranged in surface to surface contact with the second absorbent structure;
  wherein the first absorbent layer, second absorbent layer and cover cooperate to define a plurality of longitudinally extending gutters in the body facing surface of the article;
  wherein the absorbent article has a thickness of less than 4.0 mm; and
  wherein the absorbent article has an RIF value greater than 2.6.

2. The absorbent article according to claim 1, wherein the material-free zones extend over a surface area in the range of between about 50 mm$^2$ and about 4000 mm$^2$.

3. The absorbent article according to claim 1 or claim 2, wherein the plurality of material-free zones comprise between about 1 and about 7 longitudinally extending material-free zones.

4. The absorbent article according to claim 3, wherein each one of the longitudinally extending material-free zones has a length as measured along a path of the material-free zone in the range of about 5 cm to about 40 cm and a width in the range of between about 1 mm and about 10 mm.

5. The absorbent article according to any preceding claim, wherein said gutters have a depth relative to body facing surface of about 0.5 mm to about 3 mm.

6. The absorbent article according to any preceding claim, wherein the absorbent article has a thickness in the range of about 1.5 mm to about 3.0 mm.

7. The absorbent article according to any preceding claim, wherein the absorbent article has an MCB value less than 400 g, preferably less than 350 g, most preferably less than 300 g.

8. The absorbent article according to any preceding claim, wherein the absorbent article has a LTWR value greater than 1.5, preferably greater than 2.0, most preferably greater than 2.4.

9. The absorbent article according to any preceding claim, wherein the absorbent article has an RIF value greater than 2.75, preferably greater than 2.85.

10. The absorbent article according to any preceding claim, further comprising a transfer layer, the transfer layer comprising a plurality of beams and a plurality of material-free zones, each one of the material-free zones being arranged between adjacent beams, wherein each of the beams of the transfer layer are arranged in vertical alignment with a corresponding beam of the first absorbent layer and wherein each of the material-free zones of the of the transfer layer is arranged in vertical alignment with a corresponding material-free zone of the first absorbent layer.

11. An absorbent article comprising:

   a cover layer having a body facing surface;
   a first absorbent layer having an upper surface and a lower surface, the layer comprising a plurality of material-free zones, each of the material-free zones extending from the upper surface to the lower surface;
   a second absorbent structure arranged below the first absorbent system;

   wherein the cover layer includes a plurality of first regions arranged in spaced relationship to the second absorbent structure and a plurality of second regions, each of the second regions arranged in surface to surface contact with the second absorbent structure;
   wherein the absorbent article has a thickness of less than 4.0 mm; and
   wherein the absorbent article has an LTWR value greater than 2.4.

**Fig. 1**

**Fig. 2**

**Fig. 3a**

**Fig. 3b**

**Fig. 4**

**Fig. 5**

**Fig. 6**

EP 2 055 280 A2

**Fig. 7**

**Fig. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 60983780 A
- US 4555430 A
- US 5916670 A
- US 4687478 A
- US 4589876 A
- US 4900320 A
- US 4608047 A